# EUROPEAN PATENT SPECIFICATION

(11) **EP 3 313 370 B1**
(45) Date of publication and mention of the grant of the patent: **30.12.2020**
(21) Application number: 16733411.9
(22) Date of filing: 24.06.2016
(51) Int. Cl.: A61K 9/00

(54) **OPHTHALMOLOGIC PHARMACEUTICAL COMPOSITION**
PHARMAZEUTISCHE ZUBEREITUNG ZUR ANWENDUNG AM AUGE
COMPOSITION OPHTALMIQUE

(30) Priority: 26.06.2015 EP 15173997
(43) Date of publication of application: 02.05.2018
(73) Proprietor: Contipro A.S., 561 02 Dolní Dobrouc (CZ)
(72) Inventor: KALKBRENNER, Hans, 85591 Vaterstetten (DE)
(74) Representative: Dvorakova, Martina
(86) International application number: PCT/EP2016/064653
(87) International publication number: WO 2016/207340

(56) References cited:
- WO-A2-2013/171764
- US-A1- 2013 136 784
- US-A1- 2015 157 463
- JI Y ET AL: "Electrospun three-dimensional hyaluronic acid nanofibrous scaffolds", BIOMATERIALS, ELSEVIER SCIENCE PUBLISHERS BV., BARKING, GB, vol. 27, no. 20, 1 July 2006 (2006-07-01), pages 3782-3792, XP027951362, ISSN: 0142-9612 [retrieved on 2006-07-01]
- PRICE ET AL: "Hyaluronic acid: the scientific and clinical evidence", JOURNAL OF PLASTIC, RECONSTRUCTIVE AND AESTHETIC SURGERY, CHURCHILL LIVINGSTONE, GB, vol. 60, no. 10, 1 October 2007 (2007-10-01), pages 1110-1119, XP022371955, ISSN: 1748-6815, DOI: 10.1016/J.BJPS.2007.03.005

## Description

### FIELD OF THE INVENTION

The present invention is on the field of ophthalmology, in particular dosage forms of ophthalmologic drugs used for treating eye conditions, which are directly administered to the eye.

### BACKGROUND OF THE INVENTION

In pharmaceutical applications, it is essential that the active ingredients such as medicaments are applied to a specific site or via a specific route to achieve the desired effect. Moreover, the selection of the dosage regimen is of critical importance to ensure that the active ingredient is administered in the correct amount. Due to the uniqueness of eyes in terms of anatomic structure and physiology, delivering drugs to the eyes for the treatment of ophthalmologic disorders and diseases has been a challenge for a long time.

Currently, essentially all of the ophthalmologic drugs are manufactured in form of eye drops. However, this administration form has drawbacks in that it is difficult to ensure that the therapeutic agent(s) contained therein are retained on the cornea for a sufficient time and uptake and efficiency is influenced by a variety of factors, such as conjunctival blood flow, lymphatic clearance and tear dilution.

Another major problem is that eye-drops are usually self-administered by the patient. While this is convenient in that it allows the use at home, it is disadvantageous in that administering the correct dosage, i.e. the correct number of drops, may be difficult and that self-administration may in itself be a problem for patients, in particular the elderly. Thus, the eye is at risk of receiving a massive and unpredictable dose of medication or none at all.

Hence, there exists still need in the art for alternative dosage forms of ophthalmologic medications that overcome at least some of the drawbacks of existing dosage forms.

### SUMMARY OF THE INVENTION

The drawbacks resulting from the state-of-the art solutions are overcome by the pharmaceutical composition as defined in Claims. The present invention is based on the unexpected finding that pharmaceutical compositions comprising a solid carrier in form of water-soluble fibers, which readily dissolve and thereby release a therapeutically active agent to the eye. This is due to the fact that the administration of such a solid carrier is comparably simple and, as the solid carrier only dissolves and releases the therapeutically active agent once it gets into contact with the eye, it can be ensured that the correct amount is delivered.

The safe release of the therapeutically active agent to the eye and fast dissolution of the solid carrier are preferably achieved by providing pharmaceutical compositions comprising a solid carrier in form of water-soluble fibers that comprises, consists essentially of or consists of hyaluronic acid or a salt or derivative thereof.

Hence, these novel pharmaceutical compositions are ideally suited for all kinds of applications, especially those where patients administer drugs without supervision by a medical professional.

Thus, in a first aspect the present invention relates to a pharmaceutical composition for use in the treatment or prevention of ophthalmologic diseases or disorders, wherein the pharmaceutical composition comprises, consists essentially of or consists of a solid carrier in form of a non-woven or woven made of water-soluble fibers and at least one therapeutically active agent, wherein said solid carrier is impregnated with said at least one therapeutically active agent and wherein the solid carrier disintegrates upon contact with the eye.

In various embodiments, the solid carrier disintegrates upon contact with the eye within a time period of ≤ 10 seconds, preferably ≤ 5 seconds, more preferably ≤ 2 seconds, most preferably ≤ 1 second.

In various embodiments, the water-soluble fibers comprise, consist essentially of or consist of nanofibers.

The water-soluble fibers or nanofibers may comprise, consist essentially of or consist of hyaluronan (hyaluronic acid) or a pharmaceutically acceptable salt thereof. In various embodiments, the water-soluble fibers, preferably nanofibers, comprise at least 40 wt.-% hyaluronan or salt thereof, preferably 50 to 100 wt.-% hyaluronan or salt thereof.

In various embodiments, the water-soluble fibers, preferably nanofibers, comprise 40 to 95 wt.-% hyaluronan or salt thereof and optionally 5 to 60 wt.-% of at least one water-soluble hyaluronan derivative, preferably selected from the group consisting of propinylamino hyaluronan, azidyl hyaluronan, palmitoyl hyaluronan, formyl hyaluronan, caproyl hyaluronan, palmitoyl formyl hyaluronan, oleyl hyaluronan, octanoyl formyl hyaluronan, linolenoyl hyaluronan, heptanoyl formyl hyaluronan, caproyl formyl hyaluronan, butanoyl hyaluronan, anhydroformyl hyaluronan and pharmaceutically acceptable salts thereof.

In various further embodiments, the water-soluble fibers comprise hyaluronan or a salt thereof, for example in amounts of at least 40 wt.-%, preferably 40 to 95 wt.-%, and the remainder, i.e. 5 to 60 wt.-%, being one or more other water-soluble, pharmaceutically acceptable polymers other than hyaluronan and hyaluronan derivatives, such as polyethylene glycol (PEG), dextran, polyvinyl alcohol, polyvinyl pyrrolidone and the like.

In various embodiments, the salt of hyaluronan or hyaluronan derivative used may be an alkali metal salt, preferably the sodium salt.

In various embodiments of the invention, the presently claimed pharmaceutical composition comprises, consists essentially of or consists of a solid carrier that comprises at least 10 wt.-%, preferably at least 40 wt.-%, or even more preferably at least 80 wt.-% hyaluronic acid or a salt or derivative thereof. In some embodiments, the solid carrier may comprise or consists of at least 90 or 100 wt.-% hyaluronan or a salt or derivative thereof.

To facilitate administration and handling the solid carrier may have the form of a strip (for example similar to a test strip used for urine or blood tests) and may be attached to a handle or tip (for example similar to a test strip used for urine or blood tests).

In one embodiment, the at least one therapeutically active agent is selected from the group consisting of prostaglandin and prostaglandin analogues, such as Latanoprost, Dorzolamid, Bimatoprost, Tafluprost, Travoprost, and/or carbonic anhydrase inhibitors, such as Brinzolamid, and/or imidazolines, such as Brimonidin, and/or beta-blockers, such as Timolol, and/or antibiotics, such as Ofloxacin, Gentamicin, Dexamthason, Neomycin, and/or anti-histamines, such as Azelastin, Levocabastin, Ketotifen, and/or adrenergic alpha agonists, such as Tetryzolin, and/or antiseptics, such as Bibrocathol, and/or anti-inflammatory drugs, such as Prednisolon, Cromoglicic acid, Diclofenac, Ketorolac, and/or Carbomer and Povidon. Also encompassed are combinations of two or more of the listed agents.

In various embodiments, the ophthalmologic disease or disorder is selected from the group consisting of glaucoma, dry eye syndrome, macular degeneration, infection, inflammation and allergy.

In a further aspect, the invention relates to an ophthalmologic pharmaceutical composition comprising, consisting essentially of or consisting of a solid carrier in form of a non-woven or woven made of water-soluble fibers and at least one therapeutically active agent, wherein the solid carrier is impregnated with said at least one therapeutically active agent, wherein the water-soluble fibers preferably comprise, consist essentially of or consist of nanofibers and comprise, consist essentially of or consist of or hyaluronan or a pharmaceutically acceptable salt thereof and wherein the solid carrier disintegrates upon contact with the eye, preferably within a time period of ≤ 10 seconds, more preferably ≤ 5 seconds, even more preferably ≤ 2 seconds, most preferably ≤ 1 second.

In various embodiments of said compositions, the nanofibers are as defined above.

In a still further aspect, the present invention also covers the use of a non-woven or woven made of water-soluble fibers comprising, consisting essentially of or consisting of nanofibers comprising, consisting essentially of or consisting of hyaluronan or a pharmaceutically acceptable salt thereof as a solid carrier for at least one therapeutically active agent in an ophthalmologic pharmaceutical composition, wherein the non-woven or woven completely disintegrates upon contact with the eye, preferably within a time period of ≤ 10 seconds, more preferably ≤ 5 seconds, even more preferably ≤ 2 seconds, most preferably ≤ 1 second.

### BRIEF DESCRIPTION OF THE DRAWINGS

The invention will be better understood with reference to the detailed description when considered in conjunction with the accompanying drawings.

In **Figure 1** an embodiment where the solid carrier is attached to a handle is schematically depicted. In detail, the solid carrier (1) is attached to a handle (2), which can be disposable or re-usable.

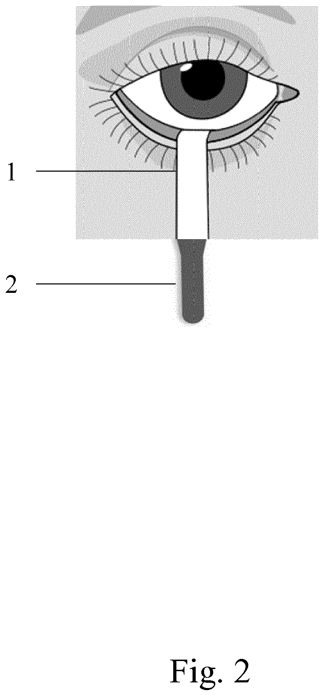

In **Figure 2** it is schematically depicted how the patient can apply the solid carrier to the eye without touching it via using the handle (2). Upon contact with the aqueous tear fluid in the eye the solid carrier dissolves and releases the therapeutically active agent.

### DETAILED DESCRIPTION OF THE INVENTION

The present invention is based on the surprising finding that eye medication may be readily administered without the risk of spilling or overdosing by use of a solid administration form, namely a solid carrier made of fibers in form or a non-woven or woven fabric. The fibers readily and rapidly dissolve upon contact with an aqueous medium, such as the tear fluid of the eye, and release an active agent with which they have been impregnated during manufacture. The application of the solid carrier, which is typically in form of a strip, is comparably easy and due to the rapid dissolution kinetics of the fibers surprisingly comfortable. This behaviour makes the solid carrier ideally suited for delivering specific doses of said active agent to the eye. The fact that the solid carrier only dissolves once it comes into contact with the moist surface of the eye, prevents premature release of the active agent and ensures correct dosing.

As described above, the present invention is in a first aspect directed to a pharmaceutical composition for use in the treatment of ophthalmologic diseases or disorders, wherein the pharmaceutical composition comprises a solid carrier in form of a non-woven or woven made of water-soluble fibers impregnated with at least one therapeutically active agent and wherein the solid carrier disintegrates upon contact with the eye.

As used herein the term "solid carrier" refers to a pharmaceutical acceptable substance that delivers the active ingredient to the site of treatment. The carrier used in accordance with the invention is solid at standard pressure and temperature (20°C, 1013 mbar) and is provided in form of fibers that form a non-woven or woven fabric. The fibers are water-soluble in that they readily dissolve upon contact with an aqueous solution and thus facilitate disintegration of the solid carrier. Preferably, the fibers completely dissolve upon contact with an aqueous solution.

As used herein the term "non-woven" refers to a web that has a structure of individual fibers or threads that are interlaid, but not in any regular, repeating manner. Non-woven webs may be formed by a variety of processes such as, for example, meltblowing processes, spunbonding process, bonded carded web processes and in relation to nanofibers by electrospinning, electroblowing and electrospraying.

Similarly, the term "woven" refers to a fabric formed of individual fibers or threads that are interlaid in a regular repeating manner.

As used herein the term "disintegrates" refers to the feature that the internal structure of the solid carrier, which is in form of fibers that form a woven or non-woven fabric, disassembles, typically by dissolution (of the fibers that make up the solid carrier) in a solvent to such an extent that it is no longer felt or noticed by the subject to that it was administered. This disintegration involves that the water-soluble fibers disassemble and/or separate and/or dissolve such that the carrier loses its structural integrity and is no longer perceptible as a foreign (solid) object by the subject. Preferably, 50 % or more, more preferably 70 % or more, most preferably 90 % by weight or more of the fibers are hydrated, moisturized or dissolved when the solid carrier is disintegrated.

In various embodiments, "disintegrates", in the sense of the present invention, means that the present solid carrier disassembles into smaller particles. These particles have a size of 10 µm or less, 5 µm or less, 2 µm or less, or 1 µm or less. The disassembly may only occur in those parts of the solid carrier that are contacted with the solvent, for example water or a moist surface like the eye.

Disintegration may, for example, be measured by contacting the solid carrier with a solvent for a predetermined amount of time, separating the solvent and the solid carrier, for example by filtration with a predetermined pore size, and determining the amount of dissolved fiber material in the filtrate or determining the remaining amount of solids retained by the filter (for example gravimetrically).

Alternatively, dissolution can be monitored by measuring turbidity of a solvent/solid carrier mixture over time. The turbidity may be measured by using a photometer. Different wavelengths may be used to measure the absorption of the solid carrier. For example, if the solid carrier contains hyaluronic acid, a wavelength of 406 nm, 450 nm or 540 nm may be used in photometrical measurements.

In various preferred embodiments, the method used to determine disintegration and dissolution of the solid carrier is a method adapted from methods used for determining dissolution properties of water-soluble films used for packaging, such as those described under the MSTM 25 of the company Monosol as of March 31, 2003. According to such an adapted method, the solid carrier is held stationary in a suitable fixture and immersed in a beaker of water at a specified temperature. The time taken for the solid carrier to disintegrate and dissolve is measured and recorded. For the testing the solid carrier is fixed to a fixture, such as a clamping device, and immersed in a beaker with 500ml of distilled water that is not stirred. The depth adjustment of the clamping device should be set so that when dropped, the end of the clamp fixed to the solid carrier will be 0.6 cm below the surface of the water. In one motion, the clamped solid carrier is dropped into the water and the timer started. Disintegration occurs when the solid carrier breaks. Dissolution occurs when all solid carrier fragments are no longer visible and the solution becomes clear. If not indicated otherwise, the dissolution properties referred to herein are determined according to this method.

"Contacted with solvent", as used herein, means the direct contact with the solvent or contact to the solvent by adhesion forces within the solid carrier. In various embodiments, at least 70 %, at least 80 %, at least 90 %, at least 95 %, at least 98 % or 100 % by weight of the solid carrier (contacted with the solvent) are disintegrated within 10 seconds. In more preferred embodiments, at least 70 %, at least 80 %, at least 90 %, at least 95 %, at least 98 % or 100 % by weight of the solid carrier (contacted with the solvent) are disintegrated within 5 seconds, preferably 2 seconds, more preferably 1 second. This degree of disintegration only relates to areas of the solid carrier that have been contacted with the solvent (directly or indirectly by adhesion). For example, 2 cm of a 5 cm strip of a pharmaceutical composition of the present invention are contacted with the eye and this 2 cm area that has been contacted with the eye disassembles within 1 second after the contact into fragments that are smaller than 2 µm and the 3 cm area that has not been contacted with the eye remains solid. In the sense of the present invention, this is considered as 100 % disintegration (if the remaining particle size may not be bigger than 2 µm), even when the 3 cm area of the strip remains solid, as the disintegration rate only relates to areas of the solid carrier that have been contacted with the solvent, for example water, the eye, a buffer solution etc.

Accordingly, as used herein, the terms "disintegrate" and "dissolve" are used to refer to the process, in which the internal structure of the solid carrier first breaks or disassembles (disintegration), for example into smaller particles, and then further break down until the fragments are no longer visible (dissolution). All references to dissolution/disintegration refer to standard conditions, i.e. 20°C and 1013 mbar and distilled water as the solvent if not explicitly indicated otherwise.

"Completely dissolved", as used herein, means that the solid carrier, upon contact with an aqueous medium, disintegrates and that the water-soluble fibers dissolve to an extent that the individual molecules making up the fiber become hydrated, e.g. in the case of water-soluble fibers comprising or consisting of hyaluronan, the fibers disintegrate into individual hyaluronan molecules. In various embodiments, the complete dissolution entails that 50 % or more, more preferably 70 % or more, most preferably 90 % or more of the water-soluble fibers are disassembled into individual molecules.

In various embodiments, upon contact with water or a moist surface, such as the eye, the solid carrier disintegrates within a time period of ≤ 10 seconds, preferably ≤ 5 seconds, more preferably ≤ 2 seconds, most preferably ≤ 1 second. This is advantageous because due to the fast disintegration of the solid carrier the active agent of the pharmaceutical composition can be easily self-administered even by untrained patients or elder patients with slight tremors, who would usually have difficulties applying eye medications such as eye drops. In especially preferred embodiments, the solid carrier disintegrates immediately upon contact with an aqueous medium, such as the tear fluid of the eye (usually wetting the cornea), i.e. the disintegration time is ≤ 1 second, preferably ≤ 0.5, 0.1, 0.05, 0.02 or 0.01 seconds.

In various embodiments, upon contact with water or a moist surface, such as the eye, the solid carrier completely dissolves within a time period of ≤ 10 seconds, preferably ≤ 5 seconds, more preferably ≤ 2 seconds, most preferably ≤ 1 second. In especially preferred embodiments, the solid carrier dissolves immediately upon contact with an aqueous medium, such as the tear fluid of the eye, i.e. the dissolution time is ≤ 1 second, preferably ≤ 0.5, 0.1, 0.05, 0.02 or 0.01 seconds.

This fast disintegration/dissolution kinetics may be due to the fast disintegration/dissolution of the water-soluble fibers that in turn also cause the solid carrier made thereof to dissolve rapidly. Accordingly, in various embodiments, the solid carrier is made of water-soluble fibers that upon contact with water disintegrate within a time period of ≤ 10 seconds, preferably ≤ 5 seconds, more preferably ≤ 2 seconds, most preferably ≤ 1 second. In the most preferred embodiments, the disintegration time is ≤ 1 second, preferably ≤ 0.5, 0.1, 0.05, 0.02 or 0.01 seconds. The water-soluble fibers preferably dissolve or completely dissolve within the afore-mentioned time periods.

Such embodiments of immediate disintegration/dissolution of the solid carrier are especially preferred, since application of the active agent to the eye is then almost instantaneous and does not cause any pain, irritation or discomfort for the patient. In other words, as soon as the person receiving the pharmaceutical composition blinks his/her eye(s) the active ingredient is already administered. This is especially advantageous for people experiencing difficulties in squeezing the vials of conventional eye drops or for those with tremors such as patients with weak hands and/or with neurodegenerative disorders such as Parkinson's disease.

Moreover, the pharmaceutical composition described herein is highly advantageous, as it allows a definite feedback about whether or not the active ingredient was applied to the eye. If administration has taken place the solid carrier is dissolved/disintegrated, at least partially. If, for instance a person with a tremor, has missed the eye, the solid carrier is still intact and hence the patient may retry administration.

Using the pharmaceutical compositions described herein, a specific and well-defined dose of an active ingredient may be delivered to the eye.

To achieve the desired dissolution kinetics upon contact with minute amounts of water, such as the moist surface of the cornea of the eye, the fibers making up the woven or non-woven of the saolid carrier are preferably nanofibers. As used herein the term "nanofiber" refers to fibers with a thickness, i.e., a diameter, of 50-1200 nm. The nanofibers can be arranged in all known ways, for example randomly or ordered in that they are arranged essentially parallel to each other or layered such that the fibers are arranged in layers that are essentially perpendicular relative to each other (with respect to the longitudinal axis of the fibers in the respective layer). Depending on the way the nanofibers are ordered, the textile strength of a nanofiber nonwoven can range from 2000-14000 MPa.

The advantages of nanofibers are small fiber diameter, very large specific surface and a high amount of small inter-fiber pores. This leads to a high reactivity of the nanofibers due to their small dimension resulting in fast release and short diffusion pathways of incorporated active ingredients and in a fast degradation of the solid nanofiber carrier.

In various embodiments, the release profile of the active ingredient may be changed by altering the structure of the nanofibrous material.

In a preferred embodiment, the solid carrier is a water-soluble non-woven that consists of nanofibers.

In various embodiments, the nanofiber non-woven has a mass per area from 0.5-200 g/m², preferably 1-10 g/m², more preferably 1.5-5 g/m² and most preferably 2.5-3.5 g/m².

While the solid carrier can be provided in various sizes and shapes, selected based on the specific application, it typically has the form of a strip of a dimension that make it suitable for application to the eye. Such a strip is, in various embodiments, of roughly rectangular shape with a length of 1 to 6 cm, preferably 1.5 to 4 cm, a breadth of 1 to 15 mm, preferably 3 to 10 mm and a thickness of 1 to 1000 µm, preferably 10 to 500 µm. The solid carrier retains the active ingredient until it disintegrates upon contact with an aqueous solution, i.e. the tear fluid in the eye. In various embodiments, the solid carrier has a mass per area of 1-10 g/m², preferably 1.5-5 g/m² and more preferably 2.5-3.5 g/m².

A solid carrier in the form of a water-soluble non-woven made of water-soluble fibers has the advantage that the solid carrier can dissolve very rapidly in a matter of seconds. This effect is particularly pronounced, if the solid water-soluble non-woven carrier consists of nanofibers. Thus, a possibly discomfort experienced by the patient upon applying the pharmaceutical composition ceases very quickly. In such a scenario, the initial discomfort is then merely a confirmation for the patient that the pharmaceutical composition was applied correctly.

In a preferred embodiment, the water-soluble fibers, preferably nanofibers, making up the woven or non-woven of the solid carrier comprise or consist of hyaluronan.

The terms "hyaluronan" and "hyaluronic acid" and "HA" are used interchangeably herein and all relate to an anionic, non-sulfated glycosaminoglycan distributed widely throughout connective, epithelial, and neural tissues that is made of D-glucuronic acid and D-N-acetylglucosamine monomers linked via alternating β-1,4 and β-1,3 glycosidic bonds. It generally is a polymer of formula (I):

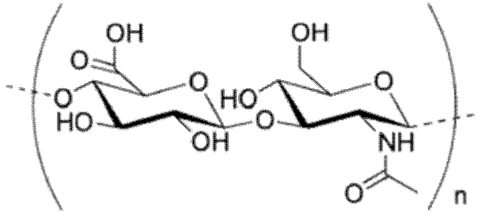

It is unique among glycosaminoglycans in that it is non-sulfated, is produced in the plasma membrane instead of the Golgi, and can be very large, with its molecular weight often being in the million Da range. As one of the major components of the extracellular matrix, hyaluronan contributes significantly to cell proliferation and migration.

Hyaluronan molecules can, for example, be 25,000 disaccharide repeats in length, i.e. n in Formula (I) can be ≤ 25,000. Hyaluronan can range in size from 5,000 to 20,000,000 Da *in vivo.* The average molecular weight in human synovial fluid is 3-4 million Da, and hyaluronan purified from human umbilical cord is about 3,000,000 to 3,200,000 Da.

In various preferred embodiments of the invention, the solid carrier comprises at least 20 wt.-%, 30 wt.-%, 40 wt.-%, 45 wt.-%, 50 wt.-%, 55 wt.-%, 60 wt.-%, 65 wt.-%, 70 wt.-%, 75 wt.-%, 80 wt.-%, 85 wt.-%, 90 wt.-% or 95 wt.-% hyaluronan in form of water-soluble fibers. In still other various embodiments, the solid carrier comprises at least 5 wt.-%, 10 wt.-%, 15 wt.-%, 20 wt.-%, 25 wt.-%, 30 wt.-%, 35 wt.-%, 40 wt.-%, 45 wt.-%, 50 wt.-%, 55 wt.-% or 60 wt.-% of at least one water-soluble hyaluronan derivative in form of water-soluble fibers. In various embodiments, the above described amounts for hyaluronan and the at least one water-soluble hyaluronan derivative can be combined in a single type of solid carrier in form of water-soluble fibers. Accordingly, in various embodiments, the fibers or nanofibers comprise 40 to 95 wt.-% hyaluronan and/or 5 to 60 wt.-% of at least one water-soluble hyaluronan derivative. In various other preferred embodiments of the invention, the fibers or nanofibers comprise at least 20 wt.-%, 30 wt.-%, 40 wt.-%, 45 wt.-%, 50 wt.-%, 55 wt.-%, 60 wt.-%, 65 wt.-%, 70 wt.-%, 75 wt.-%, 80 wt.-%, 85 wt.-%, 90 wt.-% or 95 wt.-% hyaluronan. In still other various embodiments, the fibers or nanofibers comprise at least 5 wt.-%, 10 wt.-%, 15 wt.-%, 20 wt.-%, 25 wt.-%, 30 wt.-%, 35 wt.-%, 40 wt.-%, 45 wt.-%, 50 wt.-%, 55 wt.-% or 60 wt.-% of at least one water-soluble hyaluronan derivative. In various embodiments, the above described amounts for hyaluronan and the at least one water-soluble hyaluronan derivative can be combined in a single type of fiber or nanofiber. In various other embodiments, the fibers or nanofibers consist of hyaluronan or a salt thereof, i.e. do not contain any other material or hyaluronan derivative.

In various embodiments where the solid carrier (further) comprises water-soluble hyaluronan derivatives, those are preferably selected from the group consisting of propinylamino hyaluronan, azidyl hyaluronan, palmitoyl hyaluronan, formyl hyaluronan, caproyl hyaluronan, palmitoyl formyl hyaluronan, oleyl hyaluronan, octanoyl formyl hyaluronan, linolenoyl hyaluronan, heptanoyl formyl hyaluronan, caproyl formyl hyaluronan, butanoyl hyaluronan, anhydroformyl hyaluronan and pharmaceutically acceptable salts thereof. Particularly preferred water-soluble hyaluronan derivatives are sodium palmitoyl hyaluronate sodium caproyl hyaluronate, sodium palmitoyl formyl hyaluronate, sodium oleyl hyaluronate, sodium octanoyl formyl hyaluronate, sodium linolenoyl hyaluronate, sodium heptanoyl formyl hyaluronate, sodium caproyl formyl hyaluronate, sodium butanoyl hyaluronate, sodium anhydroformyl hyaluronate. This is the case as these derivatives are especially suited for preparing solid carriers for biologically active substances.

In various embodiments the solid carrier comprises in addition to hyaluronic acid or salt thereof and/or the at least one hyaluronan derivative at least one further water-soluble polymer that is different from the hyaluronan and derivatives thereof. These additional water-soluble polymers can include polymers that carry negatively or positively charged groups along the polymer chain. Preferred water-soluble polymers are polyethylene glycol (PEG), dextran, polyvinyl alcohol, and polyvinyl pyrrolidone. In various embodiments, the pharmaceutical composition comprises a combination of hyaluronic acid and polyethylene glycol. In these combinations, the amount of other water-soluble polymer, such as PEG, is preferably 5-60 wt. %, more preferably 15-30 wt. % and even more preferably 20 wt. % relative to the total weight of the solid carrier. In such embodiments, the remainder may be the hyaluronan or a salt thereof and/or a hyaluronan derivative, as described above, with hyaluronan or salt thereof preferably making up at least 40 wt.-% of the total weight of the solid carrier. In various embodiments, the solid carrier therefore comprises 5-60 wt. %, more preferably 15-30 wt. % and even more preferably 20 wt. % relative to the total weight of the solid carrier of a water-soluble polymer different from hyaluronan or hyaluronan derivative, preferably PEG, and at least 40 to 95 wt.-%, preferably 70 to 85 wt.-%, even more preferably about 80 wt.-% of hyaluronan (or salt thereof) or of a mixture of hyaluronan and at least one hyaluronan derivative.

If the hyaluronan or hyaluronan derivative are used in form of salts, the salt may be an alkali metal or an alkaline earth metal salt, preferably the sodium salt.

Water-soluble fibers, preferably nanofibers, comprising hyaluronan and at least one hyaluronan derivative and/or other water-soluble polymer, as described above, have the advantage that they can be tailored towards the specific needs of different applications via altering parameters such as rate of disintegration upon contact with aqueous solution, porosity and anti-adhesion properties. Also the surface characteristics, which may be relevant for covalent attachment of compounds or agents or cross-linking may thus be controlled. Depending on the type and the amount of the employed hyaluronan derivatives and/or other water-soluble polymers the solubility of the nanofibers and hence of the solid carrier can be optimized for a given application. Moreover, also the release profile for the active ingredient may be changed by altering the type of hyaluronan or the hyaluronan/ hyaluronan derivative/other water-soluble polymer ratio.

In the above-described embodiments, the hyaluronan, hyaluronan derivative and other water-soluble polymer may each form separate fibers that are combined in the woven or non-woven that forms the solid carrier or two or more of said polymers may be combined in one type of fiber of the woven or non-woven that forms the solid carrier.

Examples of the above defined non-wovens or wovens made of water-soluble hyaluronan fibers, in particular nanofibers, are known in the art and have been propagated and used as scaffolds in tissue engineering or as layer in wound dressings. They are, for example, commercially available from the company Contipro (Dolni Dobrouc, Czech Republic). Said company additionally offers a variety of water-soluble hyaluronic acid based compounds that have different molecular weights and/or contain chemical modifications. A further example of a water-soluble hyaluronic acid derivative that may be used to obtain the solid carrier of the present invention is disclosed in US 8,143,391 B2.

The term "pharmaceutical composition", as used herein, refers to a composition that is made such that it is suitable for administration to animals or humans, e.g., it is made under good manufacturing practice (GMP) conditions. The composition is in solid form and may have the form of a strip. Typically, the composition is made of the solid carrier material, which is impregnated with the active agent and optionally other ingredients.

Accordingly, in various embodiments, the composition may contain in addition to the solid carrier and the active agent additional pharmaceutically acceptable excipients, such as, without limitation, diluents, surfactants, stabilizers, bulking agents, buffering substances, auxiliaries, solubilizers, binders, and chelating agents.

The diluents may, for example, comprise propylene glycol and its derivates, glycerol and its derivatives, cellulose derivatives, sucrose and its derivatives, fatty alcohols, fatty acids and its esters.

The auxiliaries may be selected from the group consisting of antioxidants, preservatives, natural or synthetic oil components, alcohols, thickening agents, vitamins and inorganic salts or combinations thereof. The inorganic salts include, but are not limited to sodium chloride, calcium chloride, magnesium chloride, and potassium chloride.

Suitable antioxidants include, without limitation, tocopherol, tocotrienol, tocomonoenol and marine tocopherol (MDT). Specifically, the antioxidant may be selected from the group consisting of alpha-, beta-, gamma-, delta- tocopherol, alpha-, beta-, gamma-, delta-tocotrienol, alpha-, beta-, gamma-, delta- tocomonoenol, alpha-, beta-, gamma-, and delta-MDT and mixtures thereof.

The pharmaceutical composition may further comprise a preservative. Useful preservatives are those that are non-irritating to the skin or the eye and which are compatible with the components of the pharmaceutical composition. Suitable preservatives include, but are not limited to sorbic acid and ethylenediamine tetraacetic acid (EDTA). In some embodiments, the preservative may include polyhexanide. The preservative may be used in a biostatic amount that prevents contamination of the composition. In an alternative embodiment, the pharmaceutical composition does not comprise a preservative. In such an embodiment, it is preferred that the pharmaceutical composition is supplied in sterile packaging, e.g. in sterile pouches or blisters. Sterilization can be carried out, e.g.by moist heat, ethylene oxide (ETO) or gamma irradiation.

If the pharmaceutical composition includes surfactants, these are preferably non-ionic. Suitable examples include polysorbate, block copolymers of ethylene oxide and propylene oxide (Pluronic®), polyethoxylated castor oil (Cremophor®), crosslinked copolymers of acrylic acid, and C10-C30 alkyl acrylate (Pemulen®).

Buffering substances that may be used include, but are not limited to Tris (Tris(hydroxymethyl)aminomethane), NaOH, histidine, tricine, lysine, glycine and serine adjusted to the correct pH with an acidic component with low ionic force.

The pharmaceutical composition may also comprise further auxiliaries selected from hypromellose (hydroxypropylmethylcellulose), chitosan, lubricin, xanthan gum, parabens, benzalkonium chloride, polyhexamethylene biguanide, arginine, glycine, heparin, sodium pentosan polysulfate, lysine and combinations thereof

The term "pharmaceutically acceptable", as used herein, refers to a substance that neither causes unacceptable loss of pharmacological activity of the active agent nor has unacceptable adverse side effects. The term thus covers agents typically used in pharmaceuticals and known to be safe for animals and humans.

The pharmaceutical composition may, once dissolved in water at 20°C, have a pH in the range of from about 6 to about 9, preferably about 7 to about 8.5 or 6 to 8, or more preferably 7.3 to 7.5 or about 7.3.

As used herein, the term "active agent" or "active ingredient" refers to a substance intended to be delivered, i.e., the substance being capable of achieving a desired action or effect. Such substances include, but are not limited to, pharmaceuticals. In alternative embodiments, the active agents may include diagnostic agents, cosmetic agents, nutritional supplements and mixtures thereof. The desired action or effect may include but is not limited to (i) a prophylactic effect on the organism, i.e. preventing an undesired biological effect such as preventing an infection, (ii) alleviating a condition caused by a disease, for example, alleviating pain or inflammation caused as a result of disease, and/or (iii) alleviating, reducing, or completely eliminating a disease from the organism. The effect may be local, or it may be systemic. In accordance therewith, "therapeutically active agent" refers to a substance that has a therapeutic effect, for example as described above, on the treated organism. The treated organisms are typically mammals, preferably humans.

Non-limiting examples of active ingredients are antibiotics, antifungals, analgesics, anaesthetics, anti-allergics, anti- inflammatory agents, and agents to treat glaucoma, e.g. prostaglandin analogues, such as Xalatan®, Lumigan®, and Travatan Z®, beta blockers, such as Timolol, alpha agonists, such as Alphagan®P and iopidine®, as well as carbonic anhydrase inhibitors.

In various embodiments, the at least one therapeutically active agent is selected from the group consisting of prostaglandin and prostaglandin analogues, carbonic anhydrase inhibitors, beta-blockers, antibiotics, anti-histamines, alpha antagonists, antiseptics, anti-inflammatory drugs, and artificial tear formulations.

In various embodiments, the at least one therapeutically active agent is selected from prostaglandin, the prostaglandin analogues Latanoprost, Dorzolamid, Bimatoprost, Tafluprost, and Travoprost, the carbonic anhydrase inhibitor Brinzolamid, the imidazoline Brimonidin, the beta-blocker Timolol, the antibiotics, Ofloxacin, Gentamicin, Dexamthason, and Neomycin, the anti-histamines Azelastin, Levocabastin, and Ketotifen, the alpha antagonist Tetryzolin, the antiseptic Bibrocathol, the anti-inflammatory drugs Prednisolon, Cromoglicic acid, Diclofenac, and Ketorolac, Carbomer and Povidon as well as combinations thereof.

In various embodiments, the active ingredient is present in the pharmaceutical composition in an effective amount. As used herein, "effective amount" means the amount of active ingredient that is sufficient to provide the desired local or systemic effect and performance at a reasonable risk/benefit ratio.

In a preferred embodiment, the pharmaceutical composition is in a single-dose form, i.e. contains the active ingredient in an amount that is sufficient for eliciting the desired therapeutic effect upon administration. This refers solely to the property that each pharmaceutical composition is for single use, i.e. that it cannot be used twice or more. It is however possible to use two or more of the single-dose compositions at about the same time (for example, one for each eye or two or more per eye to increase the dosage of the active) or at different time points over the course of a day, a week, a month, etc.

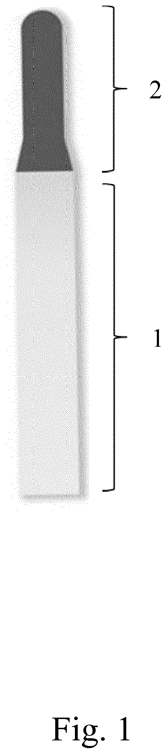

In various embodiments, the solid carrier is attached to a handle or tip. Such a handle or tip can be disposable or re-usable. The handle or tip is typically not water-soluble and therefore may have a different material composition than the solid carrier. Preferably, it is made of a commercially available plastic or paper. Alternatively, the handle may be made of the same material as the solid carrier but may be protected from dissolution during handling by a special coating. In case the solid carrier forms an essentially rectangular strip, the handle or tip may be attached on the shorter side of the solid carrier. Such type of attachment is shown in Figure 1, where a handle (2) is attached to the solid carrier. The tip or handle may also form a strip and may be as broad as the solid carrier. In various embodiments, the breadth of the tip or handle is 0.5-2.5 cm, more preferably 1.0-1.5 cm. The length of the tip or handle may preferably range from 1-3 cm, more preferably from 1.5-2 cm.

This is advantageous, because in such a case the solid carrier can be very small, as it is applied via the handle or tip, and hence does not need to be directly handled by the patients. Especially for patients with a tremor, such as the elderly, this is beneficial. Moreover, if the solid carrier is as small as possible, any potential discomfort that might be experienced upon application as well as the disintegration time of the solid carrier are minimized.

In a preferred embodiment, the active ingredient is applied to the nanofibers during their production and/or the at least one active ingredient is applied to the non-woven/woven during its production and/or the active ingredient is applied to the solid carrier after its production. The application of the active ingredient, i.e. the therapeutically active agent, may occur by impregnating the fibers, the non-woven/woven or the solid carrier with the active agent, for example in form of a powder or a solution, preferably a non-aqueous solution to prevent premature dissolution of the fibers. Such a powder or solution may be sprayed or printed onto the solid carrier, the solid carrier may be dipped or immersed in said powder or solution, or any other suitable technique for impregnating the solid carrier with the active agent may be used. It is understood, that "impregnating", as used in this context, encompasses embodiments, where the active agent is coated onto the solid carrier, as well as embodiments, where the active agent penetrates the solid carrier structure and, for example, is absorbed into pores.

While the present invention encompasses the above-described pharmaceutical compositions for ophthalmologic use as such, in various embodiments the invention is also directed to the pharmaceutical composition as described above for use in the treatment or prevention of an ophthalmologic condition, such as an ophthalmologic disease or disorder. As used herein the term "ophthalmologic condition" refers to conditions, diseases or disorders of the eye ranging from discomfort experienced by a patient without any disease to serious and non-curable diseases of the eye, including all tissues, fluids and muscles making up the eye.

As used herein the terms "disorder" and "disease" refer to states that cause pain, dysfunction, distress, social problems to the person afflicted, or similar problems for those in contact with the person including injuries, disabilities, disorders, syndromes, infections and isolated symptoms.

In various embodiments, the ophthalmologic disease or disorder is selected from the group consisting of glaucoma, retinoblastoma, conjunctivitis, dry eye syndrome, macular degeneration, infection, inflammation and allergy.

Glaucoma is a term describing a group of ocular disorders that result in optic nerve damage, often associated with increased fluid pressure in the eye (intraocular pressure). The disorders can be roughly divided into two main categories, "open-angle" and "closed-angle glaucoma. Open-angle chronic glaucoma is painless, tends to develop slowly over time and often has no symptoms until the disease has progressed significantly. It is treated with either glaucoma medication to lower the pressure, or with various pressure-reducing glaucoma surgeries. Closed-angle glaucoma, however, is characterized by sudden eye pain, redness, nausea and vomiting, and other symptoms resulting from a sudden spike in intraocular pressure, and is treated as a medical emergency. Glaucoma can permanently damage vision in the affected eye(s), first by decreasing peripheral vision (reducing the visual field), and then potentially leading to blindness if left untreated. The many different subtypes of glaucoma can all be considered to be a type of optic neuropathy. The nerve damage involves loss of retinal ganglion cells in a characteristic pattern. Raised intraocular pressure (above 21 mmHg or 2.8 kPa) is the most important and only modifiable risk factor for glaucoma. Some may have high eye pressure for years and never develop damage, a condition known as "ocular hypertension". Conversely, the term 'low tension' or 'normal tension' glaucoma is used for those with optic nerve damage and associated visual field loss, but normal or low intraocular pressure.

Dry eye syndrome is an eye disease caused by eye dryness, which, in turn, is caused by either decreased tear production or increased tear film evaporation. It is the most common eye disease, affecting 5 - 6% of the human population.

Macular degeneration (diagnosis)(MDD), often age-related macular degeneration (AMD), is a medical condition that usually affects older adults and results in a loss of vision in the center of the visual field (the macula) because of damage to the retina. Macular degeneration can make it difficult or impossible to read or recognize faces, although enough peripheral vision remains to allow other activities of daily life. In the dry (nonexudative) form, cellular debris called drusen accumulates between the retina and the choroid, causing atrophy and scarring to the retina. In the wet (exudative) form, which is more severe, blood vessels grow up from the choroid behind the retina which can leak exudate and fluid and also cause hemorrhaging. It can be treated with laser coagulation, and more commonly with medication that stops and sometimes reverses the growth of blood vessels.

In addition, the invention also relates to the use of a non-woven or woven made of water-soluble fibers comprising or consisting of nanofibers made of hyaluronan or a pharmaceutically acceptable salt thereof, as described herein, as a solid carrier for at least one therapeutically active agent in an ophthalmologic pharmaceutical composition, wherein the non-woven or woven disintegrates, dissolves or completely dissolves upon contact with the eye, preferably within the time periods disclosed above in connection with the pharmaceutical compositions.

All features and embodiments that are described in the context of the pharmaceutical composition, specifically all features and embodiments that are disclosed for the solid carrier, are herewith also explicitly disclosed for the ophthalmologic pharmaceutical composition and the use of a non-woven or woven made of water-soluble fibers.

### Examples

### Example 1: Coating of nanofibers with an active pharmaceutical ingredient (API)

This example demonstrates that nanofibers can be easily coated with a water soluble API and after dissolution of the coated layers in water the API will be retrieved in the solution. In the present example very thin layers of 10 x 20 mm (composition: 80% hyaluronic acid (82 kDa) - HA 20% polyethylene glycol (400 kDa) - PEG; Mass per area: 8 - 10 g/m²) have been used. The active pharmaceutical ingredient is Gentamicin sulfate Ph.Eur.

The experimental procedure was as follows:
1. Determination of the weight of 5 nanofiber layers.
2. Each layer is impregnated with the API by turning it in the solid powder.
3. Determination of the weight of each impregnated nanofiber layer.
4. Each of the 5 impregnated layers is put into a small glass vial and dissolved in ca. 1 ml CO₂ - free water R (Ph. Eur.).
5. Determination of the identity of the API in each of the 5 solutions using thin-layer chromatography method A, Ph.Eur. monograph Gentamicin sulfate.

**Table 1:**

| **Nanofiber layers 1x2 cm** | **1** | **2** | **3** | **4** | **5** |
|---|---|---|---|---|---|
| Weight without API (mg) | 2.20 | 3.04 | 3.23 | 2.70 | 2.33 |
| Weight impregnated with API (mg) | 6.97 | 7.13 | 5.68 | 15.30 | 4.25 |
| Amount HA in the layer (mg) | 1.76 | 2.43 | 2.58 | 2.16 | 1.86 |
| Amount PEG in the layer (mg) | 0.44 | 0.61 | 0.35 | 0.54 | 0.47 |
| Amount API (mg) | 4.77 | 4.09 | 2.45 | 12.6 | 1.92 |
| Amount Gentamicin sulfate (IE) | 3,428.7 | 2,939.9 | 1,761.1 | 9056.9 | 1,380.1 |

| Dissolution | The impregnated layers dissolved easily, instantly and completely. The solutions are turbid and not very viscous. | | | The impregnated layer dissolved easily, instantly and completely. The solution is slightly turbid and not very viscous. | The impregnated layer dissolved easily, instantly and completely. The solution is turbid and not very viscous. |
|---|---|---|---|---|---|
| Thin-layer cromatography Rf - values | 0.48 | 0.48 | 0.48 | 0.48 | 0.48 |
| Reference: | 0.60 | 0.60 | 0.60 | 0.60 | 0.60 |
| 0.48 | | | | | |
| 0.60 | | | | | |

The nanofiber layers could be easily impregnated with the API Gentamicin sulfate. The weight of the layers more than doubled after coating. Layer 4 includes more API than the others, because instead of turning the layer in the API, the API was shoveled out by the layer.

The coated layers dissolved easily, instantly and completely. The solutions were slightly turbid to turbid and not very viscous.

### Example 2: Assessment of the dissemination of the nanofibers on human eyes

This example demonstrates that nanofibers can be easily disseminated on human eyes. In the present example very thin layers of 10 x 20 mm (composition: 80 % hyaluronic acid (82 kDa) - HA; 20 % polyethylene glycol (400 kDa) - PEG; Mass per area: 3 g/m²) have been used.

The test settings were as follows: Manual application with one strip that was hold between thumb and trigger finger or by tweezers. The test subjects wore gloves during the application of the nanofiber containing strips to guarantee sterility and to avoid contact with hand sweat that could lead to premature dissolution of the test item. The strips were applied by gently wiping the ocular surface or the conjunctival sac from one side to another. The nanofibers of the strips immediately dissolved when they came into contact with the tear film. The eye was kept open by manual support due to the very bright light needed for monitoring by a camera system. The rate of dissolution/disintegration was monitored with a camera system having a time line.

The nanofiber layers were easily dissolved. The time needed to dissolve the 1,5cm strip of hyaluronic acid nanofiber was about 0.4 seconds. The high-speed camera setting confirms that the time of dissolving depends on the contact with the eye. If there is no contact, the test product does not dissolve, if there is contact with the eye surface it dissolves. This gave the patient a 100% control whether he/she touched the eye surface or not and whether the active ingredient has been delivered to the eye or not.

### Example 3: Comfort assessment of nanofiber strips compared to eye drops (ED)

This example was carried out to compare the usability and application comfort of nanofiber strips according to the present invention with that of eye drops. In this test, sodium hyaluronate microfibres (3 g/m²) designed for the targeted delivery of a superficially applied active substance into the eye have been used. The strips are compared to eye drops VISMED® (TRB Chemedica: preservative-free lubricant eye drops containing 0.18 % sodium hyaluronate from fermentation for the treatment of dryness sensations in the eye; Formulation: single dose units).

The test population was a group of male and female adult test subjects who participated on a voluntary basis and provided a signed informed consent.

Each subject had to apply the nanofiber strips of the present invention and eye drops (VISMED®) once (one test item per eye). The application of the test items on the respective eyes was performed by the subjects once without the use of a mirror.

For the experiment, the nanofiber sheet (3 g/m² HA) was cut into thin strips (∼3 mm wide and 4 cm long) by the test coordinator with the use of sterile scissors. The test subjects wore gloves during the application of the strips to guarantee sterility and to avoid contact with hand sweat that could lead to premature dissolution of the test item. The strips were applied by gently wiping the ocular surface or the conjunctival sac from one side to another. The nanofibers of the strip immediately dissolved when they came into contact with the tear film.

Single dose VISMED® was used in accordance with the provider's instructions.

Directly after the application, the subject was asked to complete a patient questionnaire comparing the usability and application comfort of the two test items. The results of these questionnaires are summarized in Fig. 4.

The overall usability of both application forms was performed after both test items have been applied. The subject was asked to evaluate the different aspects considering the main intended purpose of the strips of the present invention, namely the targeted and reliable delivery of a defined dose of a medical compound fictitiously contained therein. The detailed results are given in Figure 4. The user-friendly handling of the nanofiber strips in comparison to eye drops was evaluated as "better" by the majority of subjects (71.4 %). 21.4 % of the subjects felt no difference and 7.1 % favored the handling of eye drops.

All subjects confirmed their hypothetical willingness to use the nanofibers regularly (100 %).

The majority of subjects stated that they would prefer the nanofiber strip application form (57.1 %), while 28.6 % had no preference for either test item. 14.3 % favored eye drops.

## Claims

1. Pharmaceutical composition for use in the treatment or prevention of ophthalmologic diseases or disorders, wherein the pharmaceutical composition comprises, consists essentially of or consists of a solid carrier in form of a non-woven or woven made of water-soluble fibers and at least one therapeutically active agent, wherein the solid carrier is impregnated with said at least one therapeutically active agent and wherein the solid carrier disintegrates upon contact with the eye within a time period of ≤ 10 seconds, preferably ≤ 5 seconds, more preferably ≤ 2 seconds, most preferably ≤ 1 second, wherein the water-soluble fibers comprise at least 40 wt.-%, preferably 50 to 100 wt.-% hyaluronan (hyaluronic acid) or a pharmaceutically acceptable salt thereof.

2. Pharmaceutical composition for use according to claim 1, wherein the water-soluble fibers comprise, consist essentially of or consist of nanofibers.

3. Pharmaceutical composition for use according to claim 1, wherein the fibers comprise 40 to 95 wt.-% hyaluronan and 5 to 60 wt.-% of at least one water-soluble hyaluronan derivative, preferably selected from the group consisting of propinylamino hyaluronan,
azidyl hyaluronan, palmitoyl hyaluronan, formyl hyaluronan, caproyl hyaluronan, palmitoyl formyl hyaluronan, oleyl hyaluronan, octanoyl formyl hyaluronan, linolenoyl hyaluronan, heptanoyl formyl hyaluronan, caproyl formyl hyaluronan, butanoyl hyaluronan, anhydroformyl hyaluronan and pharmaceutically acceptable salts thereof.

4. Pharmaceutical composition for use according to any one of claims 1 to 3, wherein the salt of hyaluronan or hyaluronan derivative is an alkali metal salt, preferably the sodium salt.

5. Pharmaceutical composition for use according to any one of claims 1 to 4, wherein the solid carrier further comprises 5-60 wt. %, more preferably 15-30 wt. % and even more preferably 20 wt. % relative to the total weight of the solid carrier of a water-soluble polymer different from hyaluronan or hyaluronan derivative, preferably PEG, and at least 40 to 95 wt.-%, preferably 70 to 85 wt.-%, even more preferably about 80 wt.-% of hyaluronan or salt thereof or of a mixture of hyaluronan and at least one hyaluronan derivative.

6. Pharmaceutical composition for use according to any one of claims 1 to 5, wherein the solid carrier has the form of a strip.

7. Pharmaceutical composition for use according to any one of claims 1 to 6, wherein the solid carrier is attached to a handle or tip.

8. Pharmaceutical composition for use according to any one of claims 1 to 7, wherein the at least one therapeutically active agent is selected from the group consisting of Latanoprost, Dorzolamid, Brinzolamid, Bimatoprost, Tafluprost, Travoprost, Brimonidin, Timolol, Ofloxacin, Gentamicin, Dexamthason, Neomycin, Azelastin, Prednisolon, Tetryzolin, Bibrocathol, Cromoglicinsäure, Levocabastin, Ketotifen, Diclofenac, Ketorolac, Carbomer, Povidon and combinations thereof.

9. Pharmaceutical composition for use according to any one of claims 1 to 8, wherein the ophthalmologic disease or disorder is selected from the group consisting of glaucoma, dry eye syndrome, macular degeneration, infection, inflammation and allergy.

## Patentansprüche

1. Pharmazeutische Zusammensetzung zur Anwendung bei der Behandlung oder Vorbeugung von ophthalmologischen Erkrankungen oder Störungen, wobei die pharmazeutische Zusammensetzung einen festen Träger in der Form eines ungewebten oder gewebten, aus wasserlöslichen Fasern hergestellten Gebildes sowie wenigstens eine therapeutisch wirksame Substanz umfasst, bzw. aus derartigem Gebilde und derartiger Substanz besteht, oder im Wesentlichen besteht, wobei der feste Träger mit der besagten wenigstens einen therapeutisch wirksamen Substanz imprägniert ist und wobei sich der feste Träger beim Kontakt mit dem Auge innerhalb eines Zeitraums von ≤ 10 Sekunden, vorzugsweise von ≤ 5 Sekunden, vorzüglicherweise von ≤ 2 Sekunden, am meisten bevorzugt von ≤ 1 Sekunde zersetzt,
wobei die wasserlöslichen Fasern Hyaluronan (Hyaluronsäure) oder ein pharmazeutisch akzeptierbares Salz desselben (derselben) in einer Menge von wenigstens 40 Gew.-%, vorzugsweise von 50 bis 100 Gew.-%, umfassen.

2. Pharmazeutische Zusammensetzung zur Anwendung nach Anspruch 1, wobei die wasserlöslichen Fasern Nanofasern umfassen oder aus Nanofasern bestehen, oder im Wesentlichen bestehen.

3. Pharmazeutische Zusammensetzung zur Anwendung nach Anspruch 1, wobei die Fasern Hyaluronan in einer Menge von 40 bis 95 Gew.-% und ein wasserlösliches Derivat des Hyaluronans in einer Menge von 5 bis 60 Gew.-% umfasst, wobei das Derivat vorzugsweise aus der Gruppe ausgewählt sind, die aus Propinylamino-Hyaluronan, Azidyl-Hyaluronan, Palmitoyl-Hyaluronan, Formyl-Hyaluronan, Caproyl-Hyaluronan, Palmitoyl-Formyl-Hyaluronan, Oleyl-Hyaluronan, Octanoyl-Formyl-Hyaluronan, Linolenoyl-Hyaluronan, Heptanoyl-Formyl-Hyaluronan, Caproyl-Formyl-Hyaluronan, Butanoyl-Hyaluronan, Anhydroformyl-Hyaluronan sowie aus pharmazeutisch akzeptierbaren Salzen derselben besteht.

4. Pharmazeutische Zusammensetzung zur Anwendung nach einem der Ansprüche 1 bis 3, wobei das Salz der Hyaluronsäure oder eines Derivats der Hyaluronsäure ein Salz eines Alkalimetalls, vorzugsweise Natriumsalz ist.

5. Pharmazeutische Zusammensetzung zur Anwendung nach einem der Ansprüche 1 bis 4, wobei der feste Träger ferner ein von Hyaluronan sowie von Derivaten der Hyaluronsäure sich unterscheidendes wasserlösliches Polymer, vorzugsweise PEG, in der Menge von 5-60 Gew.-%, vorzugsweise 15-30 Gew.-%, vorzüglicherweise 20 Gew.-%, bezogen auf das Gesamtgewicht des festen Trägers, sowie Hyaluronan oder dessen Salz oder ein Gemisch von Hyaluronan und wenigsten einem Derivat der Hyaluronsäure in der Menge von mindestens 40 bis 95 Gew.-%, vorzugsweise von 70 bis 85 Gew.-%, vorzüglicherweise von ungefähr 80 Gew.-% umfasst.

6. Pharmazeutische Zusammensetzung zur Anwendung nach einem der Ansprüche 1 bis 5, wobei der feste Träger in der Form eines Streifens vorliegt.

7. Pharmazeutische Zusammensetzung zur Anwendung nach einem der Ansprüche 1 bis 6, wobei der feste Träger an einem Handgriff oder an einer Spitze angebracht ist.

8. Pharmazeutische Zusammensetzung zur Anwendung nach einem der Ansprüche 1 bis 7, wobei die wenigstens eine therapeutisch wirksame Substanz aus der Gruppe ausgewählt ist, die aus Latanoprost, Dorzolamid, Brinzolamid, Bimatoprost, Tafluprost, Travoprost, Brimonidin, Timolol, Ofloxacin, Gentamicin, Dexamthason, Neomycin, Azelastin, Prednisolon, Tetryzolin, Bibrocathol, Cromoglicinsäure, Levocabastin, Ketotifen, Diclofenac, Ketorolac, Carbomer, Povidon sowie aus Kombinationen derselben besteht.

9. Pharmazeutische Zusammensetzung zur Anwendung nach einem der Ansprüche 1 bis 8, wobei die ophthalmologische Erkrankung oder Störung aus der Gruppe ausgewählt ist, die Glaukom, Syndrom des trockenen Auges, makuläre Degeneration, Infektion, Entzündung und Allergie besteht.

## Revendications

1. Une composition pharmaceutique pour une utilisation dans le traitement ou la prévention des maladies ou troubles ophtalmologiques, où la composition pharmaceutique comprend, consiste essentiellement en ou consiste en véhicule solide sous forme d'un non tissé ou un tissé fait de fibres solubles dans l'eau et au moins un agent thérapeutiquement actif, où le véhicule solide est imprégné dudit au moins un agent thérapeutiquement actif et où le véhicule solide se désintègre en contact avec l'œil dans une période de temps de ≤ 10 secondes, de préférence ≤ 5 secondes, mieux encore ≤ 2 secondes, idéalement ≤ 1 seconde, où les fibres solubles dans l'eau comprennent au moins 40 % en poids, de préférence de 50 à 100 % en poids d'hyaluronane (acide hyaluronique) ou un sel de celui-ci pharmaceutiquement acceptable.

2. La composition pharmaceutique pour une utilisation selon la revendication 1, où les fibres solubles dans l'eau comprennent, consistent essentiellement en ou consistent en nanofibres.

3. La composition pharmaceutique pour une utilisation selon la revendication 1, où les fibres comprennent de 40 à 95 % en poids d'hyaluronane et de 5 à 60 % en poids d'au moins un dérivé hyaluronane soluble dans l'eau, de préférence choisi dans le groupe consistant en propinylamino hyaluronane, azidyle hyaluronane, palmitoyle hyaluronane, formyle hyaluronane, caproyle hyaluronane, palmitoyle formyle hyaluronane, oléyle hyaluronane, octanoyle formyle hyaluronane, linolenoyle hyaluronane, heptanoyle formyle hyaluronane, caproyle formyle hyaluronane, butanoyle hyaluronane, anhydroformyle hyaluronane et les sels de ceux-ci pharmaceutiquement acceptables.

4. La composition pharmaceutique pour une utilisation selon l'une quelconque des revendications 1 à 3, où le sel d'hyaluronane ou le dérivé hyaluronane est un sel de métal alcalin, de préférence un sel sodique.

5. La composition pour une utilisation selon l'une quelconque des revendications 1 à 4, où le véhicule solide comprend également 5-60 % en poids, mieux encore 15-30 % en poids et idéalement 20 % en poids par rapport au poids total du véhicule solide un polymère soluble dans l'eau, différent de l'hyaluronane ou du dérivé d'hyaluronane, de préférence PEG, et au moins de 40 à 95 % en poids, de préférence de 70 à 85 % en poids, idéalement environ 80 % en poids d'hyaluronane ou d'un sel de celui-ci ou d'un mélange d'hyaluronane et au moins un dérivé hyaluronane.

6. La composition pharmaceutique pour une utilisation selon l'une quelconque des revendications 1 à 5, où le véhicule solide est sous forme d'une bande.

7. La composition pharmaceutique pour une utilisation selon l'une quelconque des revendications 1 à 6, où le véhicule est fixé à un manche ou une pointe.

8. La composition pharmaceutique pour une utilisation selon l'une quelconque des revendications 1 à 7, où ledit au moins un agent thérapeutiquement actif est choisi dans le groupe consistant en Latanoprost, Dorzolamid, Brinzolamid, Bimatoprost, Tafluprost, Travoprost, Brimonidin, Timolol, Ofloxacin, Gentamicin, Dexamthason, Neomycin, Azelastin, Prednisolon, Tetryzolin, Bibrocathol, l'acide cromoglycique, Levocabastin, Ketotifen, Diclofenac, Ketorolac, Carbomer, Povidon est des combinations de ceux-ci.

9. La composition pour une utilisation selon l'une quelconque des revendications 1 à 8, où la maladie ou le trouble ophtalmologique est choisi dans le groupe consistant en glaucome, syndrome de l'œil sec, dégénérescence maculaire, infection, inflammation et allergie.
